(19) Europäisches Patentamt
European Patent Office
Office européen des brevets

(11) Numéro de publication : **0 091 328**
**B1**

# (12) FASCICULE DE BREVET EUROPÉEN

(45) Date de publication du fascicule du brevet : **11.11.87**

(51) Int. Cl.⁴ : **C 07 D209/90**, A 61 K 31/40

(21) Numéro de dépôt : **83400414.5**

(22) Date de dépôt : **01.03.83**

(54) **Nouveaux dérivés du trans 4-amino benz (c,d) indol-5-ol ainsi que leurs sels, leur préparation, leur application comme médicaments et compositions les renfermant.**

(30) Priorité : **05.03.82 FR 8203726**

(43) Date de publication de la demande :
**12.10.83 Bulletin 83/41**

(45) Mention de la délivrance du brevet :
**11.11.87 Bulletin 87/46**

(84) Etats contractants désignés :
**CH DE FR GB IT LI NL**

(56) Documents cités :
**EP-A- 0 037 784**
**GB-A- 745 495**
**US-A- 4 238 486**
**HELVETICA CHIMICA ACTA, vol. 35, partie II, fascicules V-VIII, 1952, pages 148-151, Helvetica Chimica Acta Verlag, Basel, CH., A. STOLL et al.: "Neue Synthesen des Benz(cd)indolins"**
**JOURNAL OF THE AMERICAN CHEMICAL SOCIETY, vol. 78, no. 13, 5 juillet 1956, pages 3087-3114, American Chemical Society, USA, E.C. KORNFELD et al.: "The total synthesis of lysergic acid"**

(73) Titulaire : **ROUSSEL-UCLAF**
**35, boulevard des Invalides**
**F-75007 Paris (FR)**

(72) Inventeur : **Nedelec, Lucien**
**45, boulevard de l'Ouest**
**F-93340-Le Raincy (FR)**
Inventeur : **Brown, Neil Leslie**
**12, rue Jaucourt**
**F-75012-Paris (FR)**
Inventeur : **Plassard, Guy**
**6, rue Clément Marot**
**F-91600-Savigny-sur-Orge (FR)**

(74) Mandataire : **Niel, Michel et al**
**Département des Brevets ROUSSEL UCLAF B.P. no. 9**
**F-93230 Romainville (FR)**

EP 0 091 328 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne de nouveaux dérivés du trans 4-amino benz(c,d) indol-5-ol ainsi que leurs sels, leur préparation, leur application ainsi que de produits apparentés comme médicaments et les compositions les renfermant.

L'invention a pour objet de nouveaux dérivés racémiques ou dédoublés du trans 4-amino benz(c,d) indol-5-ol, ainsi que leurs sels d'addition avec les acides minéraux ou organiques, lesdits dérivés répondant à la formule générale (I) :

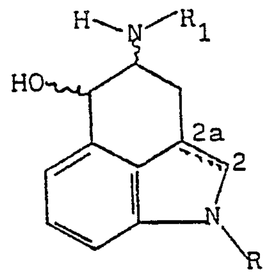

(I)

dans laquelle $R_1$ représente :

un atome d'hydrogène,

un radical alcoyle, linéaire ou ramifié, renfermant de 1 à 8 atomes de carbone, éventuellement substitué par un ou plusieurs atomes d'halogène, par un ou plusieurs radicaux hydroxy ou par un radical phényle ou phénoxy, éventuellement substitué, sur le noyau phényle par un ou plusieurs atomes d'halogène ou par un ou plusieurs radicaux hydroxy, méthyl ou méthoxy ;

un radical cycloalcoyle renfermant de 3 à 7 atomes de carbone,

un radical cycloalcoylalcoyle renfermant de 4 à 7 atomes de carbone, ou

un radical alcényle ou alcynyle renfermant de 3 à 6 atomes de carbone, le trait pointillé en 2-2a représente une éventuelle seconde liaison carbone-carbone et R représente un atome d'hydrogène.

Dans la formule générale I et dans ce qui suit, le terme radical alcoyle, linéaire ou ramifié, renfermant de 1 à 8 atomes de carbone, désigne, par exemple, un radical méthyle, éthyle, n-propyle, isopropyle, n-butyle, isobutyle ou pentyle, et de préférence un radical méthyle, éthyle, propyle, isopropyle ou isobutyle ; lorsque le radical alcoyle ou phényle est substitué par des atomes d'halogène, il s'agit de préférence d'atomes de chlore ; le radical alcoyle comporte de préférence, un seul substituant ;

le terme radical cycloalcoyle renfermant de 3 à 7 atomes de carbone, désigne, par exemple, un radical cyclopropyle, mais de préférence, un radical cyclobutyle ou cyclopentyle ; le terme radical cycloalcoylalcoyle renfermant de 4 à 7 atomes de carbone désigne, de préférence, un radical cyclopropylméthyle ; le terme radical alcényle renfermant de 3 à 6 atomes de carbone désigne, de préférence un radical allyle, le terme radical alcynyle renfermant de 3 à 6 atomes de carbone désigne, de préférence un radical propargyle ; les traits ondulés signifient que les substituants en positions 4 et 5 des dérivés de formule I, sont de configuration trans.

Bien entendu, les mélanges racémiques des dérivés de formule I, aussi bien que les dérivés dédoublés entrent dans le cadre de l'invention.

Les sels d'addition avec les acides minéraux ou organiques peuvent être par exemple, les sels formés avec les acides chlorhydrique, bromhydrique, iodhydrique, nitrique, sulfurique, phosphorique, acétique, formique, propionique, benzoïque, maléique, fumarique, succinique, tartrique, citrique, oxalique glyoxylique, aspartique, alcanesulfoniques, tels que les acides méthane ou éthanesulfoniques, arylsulfoniques, tels que les acides benzène ou paratoluène sulfoniques et arylcarboxyliques.

Parmi les dérivés, objet de l'invention, on peut citer ceux répondant à la formule I dans laquelle une simple liaison est présente en 2-2a, ainsi que leurs sels d'addition avec les acides minéraux ou organiques, on peut citer également ceux répondant à la formule I dans laquelle une double liaison est présente en 2-2a, ainsi que leurs sels d'addition avec les acides.

Parmi les dérivés, objet de l'invention, on retient de préférence ceux répondant à la formule I dans laquelle $R_1$ représente un atome d'hydrogène, un radical alcoyle, linéaire ou ramifié, renfermant de 1 à 6 atomes de carbone, éventuellement substitué, ainsi que leurs sels d'addition avec les acides.

Parmi ceux-ci, on retient notamment ceux répondant à la formule I dans laquelle $R_1$ représente un atome d'hydrogène, ou un radical alcoyle, linéaire ou ramifié non substitué renfermant de 1 à 6 atomes de carbone, ainsi que leurs sels d'addition avec les acides.

Parmi ces derniers, on retient tout particulièrement, les dérivés dont les noms suivent :

le trans 4RS 4-méthylamino 1,2,2a,3,4,5-hexahydro benz(c,d) indol-5-ol,

le trans 4RS 4-méthylamino 1,3,4,5-tétrahydro benz(c,d) indol-5-ol ainsi que leurs sels d'addition avec les acides.

L'invention a également pour objet un procédé de préparation des dérivés, tels que définis par la

formule I ci-dessus, ainsi que de leurs sels, caractérisé en ce que l'on réduit un produit de formule II :

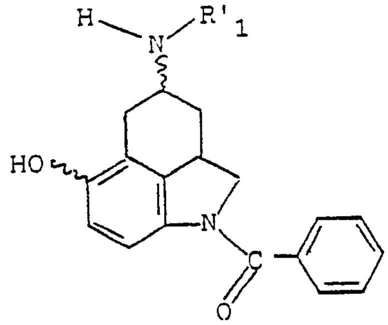

(II)

dans laquelle $R'_1$ représente un atome d'hydrogène ou un radical méthyle, pour obtenir un produit de formule $I'_A$ :

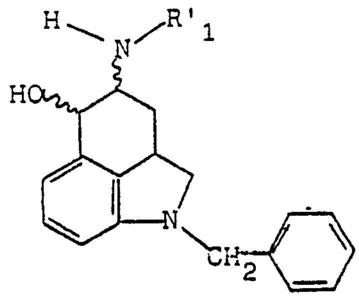

($I'_A$)

dans laquelle $R'_1$ a la signification déjà indiquée, que :
soit l'on soumet à une débenzylation, pour obtenir un produit de formule $I_A$ :

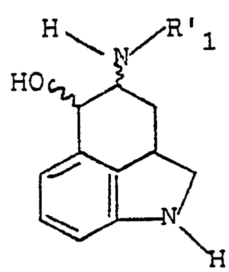

($I_A$)

dans laquelle $R'_1$ a la signification déjà indiquée, que ou bien l'on isole ou bien l'on fait réagir dans le cas où $R'_1$ représente un atome d'hydrogène, avec un dérivé carbonylé de formule III :

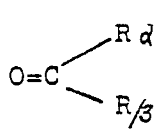

dans laquelle $R_\alpha$ et $R_\beta$ sont tels que

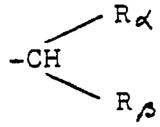

a la signification de $R_1$ déjà indiquée, à l'exception d'un atome d'hydrogène et d'un radical méthyle, puis soumet le dérivé obtenu à l'action d'un agent de réduction, pour obtenir un produit de formule $I_B$ :

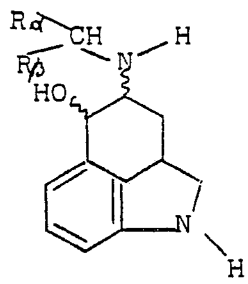

($I_B$)

dans laquelle $R_\alpha$ et $R_\beta$ ont la signification déjà indiquée que l'on isole, si désiré,

soit l'on soumet ledit produit de formule $I'_A$ à l'action d'un agent de blocage de la fonction amine, pour obtenir un produit de formule IV :

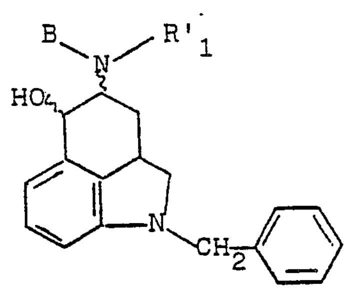

(IV)

dans laquelle B représente le groupement de blocage de la fonction amine et $R'_1$ a la signification déjà indiquée, que l'on soumet à une débenzylation, pour obtenir un produit de formule V :

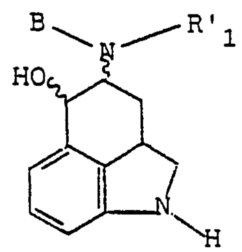

(V)

dans laquelle B et $R'_1$ ont la signification déjà indiquée, que l'on soumet à l'action d'un agent d'oxydation, pour obtenir un produit de formule VI :

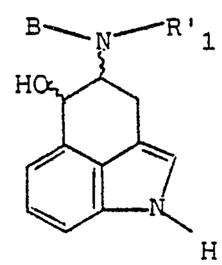

(VI)

dans laquelle B et $R'_1$ ont la signification déjà indiquée, dont on libère la fonction amine bloquée, pour obtenir un produit de formule $I_C$ :

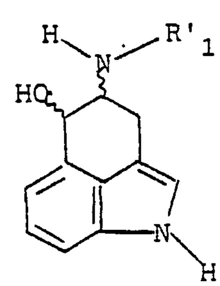

($I_C$)

que soit l'on isole soit l'on fait réagir dans le cas où $R'_1$ représente un atome d'hydrogène, avec un dérivé carbonylé de formule III définie ci-dessus, puis avec un agent réducteur pour obtenir un produit de formule $I_D$ :

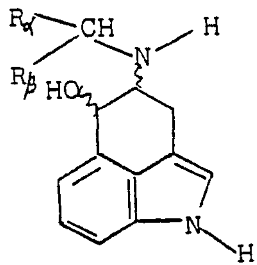

($I_D$)

dans laquelle $R_\alpha$ et $R_\beta$ ont la signification déjà indiquée ;

soit l'on fait réagir ledit produit de formule $I'_A$ dans laquelle $R'_1$ représente un atome d'hydrogène avec un dérivé carbonylé de formule III définie ci-dessus, puis avec un agent réducteur, pour obtenir un produit de formule $I'_B$ :

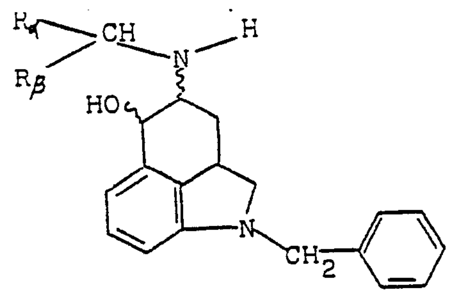

($I'_B$)

dans laquelle $R_\alpha$ et $R_\beta$ ont la signification déjà indiquée, puis, si désiré, salifie les produits de formule I obtenus au cours du procédé.

Lors de la mise en œuvre du procédé ci-dessus décrit, il est également possible d'obtenir directement le produit de formule $I_A$ à partir du produit de formule II par débenzylation directe de préférence dans ce cas, par hydrolyse acide, on utilise avantageusement un acide minéral tel que l'acide chlorhydrique à la concentration 6N par exemple. Une hydrolyse basique est également possible.

Dans des conditions préférentielles de mise en œuvre de l'invention, le procédé de préparation ci-dessus décrit est caractérisé en ce que :

la réduction du produit de formule II est réalisée à l'aide d'un hydrure mixte tel que l'hydrure d'aluminium-lithium, en présence d'un acide de Lewis tel que le chlorure d'aluminium,

la débenzylation des produits de formules $I'_A$ et IV est effectuée par hydrogénation catalytique ; on utilise, par exemple, un catalyseur à base de palladium, en présence d'acide acétique,

la réaction des produits de formules $I'_A$, $I_A$ et $I_C$ avec le dérivé carbonylé de formule III est réalisée en présence d'une base, de préférence la triéthylamine, la réaction est avantageusement réalisée dans un solvant tel que l'acétonitrile ou de préférence, un alcanol tel que le méthanol ou l'éthanol,

l'agent de réduction conduisant aux produits $I_B$, $I_D$ et $I'_B$ peut être un borohydrure ou un cyano borohydrure alcalin, notamment le borohydrure de sodium. Cette réduction est effectuée de préférence par hydrogénation catalytique,

le groupement de blocage de la fonction amine est de préférence un radical trifluoroacétyle ; on le fixe sur la fonction amine ainsi que sur la fonction alcool, par action d'anhydride trifluoroacétique ou d'un halogénure de trifluoroacétyle, puis débloque la fonction alcool sélectivement, de préférence, à l'aide d'un bicarbonate alcalin, notamment de sodium, dans un alcanol de faible poids moléculaire tel que le méthanol,

l'agent d'oxydation du produit de formule V est le bioxyde de manganèse, on peut cependant utiliser également le palladium dans le xylène au reflux ;

le déblocage de la fonction amine est réalisé par hydrolyse basique, de préférence à l'aide d'un hydroxyde alcalin, notamment de sodium.

Le produit de formule II dans laquelle $R'_1$ représente un atome d'hydrogène est décrit dans la demande de brevet français n° 2479830 ; celui pour lequel $R'_1$ représente un radical méthyle peut être préparé comme indiqué dans la demande précitée.

Le produit de formule $I'_A$ dans laquelle $R'_1$ représente un radical méthyle peut également être préparé par méthylation du produit de formule $I'_A$ dans laquelle $R'_1$ représente un atome d'hydrogène notamment par passage au carbamate de méthyle de ce dernier, par exemple, par condensation, avec un haloformiate de méthyle, de préférence le chloroformiate, puis réduction, par exemple, à l'aide d'hydrure d'aluminium-lithium.

Un exemple d'une telle préparation figure ci-après dans la partie expérimentale.

Les dérivés de formule I et I' présentent un caractère basique. On peut avantageusement préparer les sels d'addition des dérivés de formule I, et I' en faisant réagir, en proportions sensiblement stœchiométri-

ques, un acide minéral ou organique avec ledit dérivé de formule I ou I'. Les sels peuvent être préparés sans isoler les bases correspondantes.

Le dédoublement des dérivés racémiques de formule I et I' peut être réalisé selon les procédés classiques.

Les dérivés du trans 4-amino benz(c,d) indol-5-ol répondant à la formule I, $I'_A$ et $I'_B$ ainsi que leurs sels, possèdent de très intéressantes propriétés pharmacologiques ; ils sont doués en particulier de remarquables propriétés hypotensives et antihypertensives et notamment de propriétés vasodilatatrices.

Ces propriétés sont illustrées plus loin dans la partie expérimentale.

Ces propriétés justifient l'utilisation des dérivés du trans 4-amino benz(c,d) indol-5-ol répondant à la formule I, $I'_A$ et $I'_B$ ainsi que de leurs sels pharmaceutiquement acceptables à titre de médicaments.

La présente demande a ainsi également pour objet l'application, à titre de médicaments, des dérivés répondant à la formule I' :

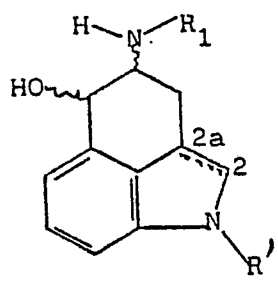

(I')

dans laquelle $R_1$ représente :

un atome d'hydrogène ;

un radical alcoyle, linéaire ou ramifié renfermant de 1 à 8 atomes de carbone, éventuellement substitué par un ou plusieurs radicaux hydroxy ou par un radical phényle ou phénoxy éventuellement substitué sur le noyau phényle par un ou plusieurs atomes d'halogène ou par un ou plusieurs radicaux hydroxy, méthyl ou méthoxy,

un radical cycloalcoyle renfermant de 3 à 7 atomes de carbone,

un radical cycloalcoylalcoyle renfermant de 4 à 7 atomes de carbone, ou

un radical alcényle ou alcynyle renfermant de 3 à 6 atomes de carbone, et soit le trait pointillé en 2-2a représente une seconde liaison carbone-carbone et R' représente un atome d'hydrogène, soit le trait pointillé en 2-2a ne représente pas une seconde liaison carbone-carbone et R' représente un atome d'hydrogène ou un radical benzyle, étant entendu que lorsque R' représente un radical benzyle, $R_1$ ne représente pas un atome d'hydrogène ainsi que de leurs sels d'addition avec les acides pharmaceutiquement acceptables.

Les composés de la formule I' ne tombant pas sous la formule I sont décrits à titre d'intermédiaires dans EP-A1-0 037 784.

Parmi les médicaments selon l'invention, on peut citer en particulier les produits préférés mentionnés précédemment et tout particulièrement :

le trans 4RS 4-méthylamino 1,2,2a,3,4,5-hexahydro benz(c,d) indol-5-ol,

le trans 4RS 4-méthylamino 1,3,4,5-tétrahydro benz(c,d) indol-5-ol,

ainsi que leurs sels d'addition avec les acides minéraux ou organiques.

Les médicaments, selon l'invention, trouvent leur emploi, par exemple, dans le traitement de l'hypertension artérielle essentielle, de l'hypertension de la cinquantaine, de la ménopause, du diabétique, de l'obèse et du pléthorique, ainsi que dans le traitement de l'hypertension artérielle du sujet âgé ou atteint d'artériosclérose dans le traitement de l'hypertension d'origine rénale et dans le traitement de l'insuffisance cardiaque.

La dose usuelle, variable selon le produit utilisé, le sujet traité et l'affection en cause, peut être, par exemple, de 5 mg à 200 mg par jour du produit de l'exemple 2, par voie orale chez l'homme.

L'invention a aussi pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule I' et leurs sels d'addition avec les acides pharmaceutiquement acceptables peuvent être incorporés dans les compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables, elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les

glycols, les divers agents mouillants, dispersants ou émulsifiants, les conservateurs.

Les exemples suivants illustrent l'invention sans toutefois la limiter.

Exemple 1 : dichlorhydrate de trans 4RS 4-amino 1,2,2a,3,4,5-hexahydro benz(c,d) indol-5-ol

On porte au reflux sous agitation, 5,88 g de trans 4RS 4-amino 1-benzoyl 1,2,2a,3,4,5-hexahydro benz(c,d) indol-5-ol avec 60 $cm^3$ de dioxanne et 60 $cm^3$ d'acide chlorhydrique 6N pendant 2 heures, refroidit, verse dans 500 $cm^3$ d'eau glacée, alcalinise par de la lessive de soude, extrait au chlorure de méthylène à 10 % de méthanol, lave à l'eau, sèche, filtre et amène à sec sous pression réduite.

On ajoute quelques $cm^3$ d'éther, essore, sèche pendant deux heures à 50 °C sous pression réduite et obtient le produit attendu.

$1^{er}$ jet : 2,73 g. F ≃ 195 °C puis $2^{ème}$ jet : 0,32 g. F = 188 °C.

Formation de dichlorhydrate

On dissout 3 g de la base ci-dessus dans 250 $cm^3$ d'éthanol, ajoute 10 $cm^3$ d'une solution éthanolique d'acide chlorhydrique 3N, essore, lave à l'éther, sèche sous vide le précipité formé et obtient 3,5 g du produit attendu. F > 250 °C.

Analyse : pour $C_{11}H_{16}N_2OCl_2$ = 263,15

| | | | | |
|---|---|---|---|---|
| Calculé : | C 50,20 | H 6,13 | N 10,64 | Cl 26,94 % |
| Trouvé : | C 50,2 | H 6,1 | N 10,3 | Cl 26,9 %. |

Exemple 2 : Fumarate neutre de trans 4RS 4-(méthylamino) 1,2,2a,3,4,5-hexahydro benz(c,d) indol-5-ol

Stade A : Trans 4RS 4-amino 1,2,2a,3,4,5-hexahydro 1-phénylméthyl benz(c,d) indol-5-ol

On ajoute lentement sous atmosphère inerte et agitation 26 g de trans 4RS 4-amino 1-benzoyl 1,2,2a,3,4,5-hexahydro benz(c,d) indol-5-ol, à 26 g d'hydrure d'aluminium lithium et 13 g de chlorure d'aluminium dans 800 $cm^3$ de dioxane, porte deux heures au reflux, refroidit au bain de glace, ajoute, goutte à goutte, 300 $cm^3$ de tétrahydrofuranne hydraté à 20 % et 300 $cm^3$ de soude 2N, essore le précipité formé, lave au chlore de méthylène, reprend le filtrat par 1,5 litre de chlorure de méthylène, lave à l'eau, sèche, évapore à sec et récupère 20 g du produit attendu fondant à 166 °C après recristallisation dans le chlorure de méthylène.

Stade B : Trans 4RS 1,2,2a,3,4,5-hexahydro 4-méthylamino 1-(phénylméthyl) benz(c,d) indol-5-ol

a) Condensation

On dissout 10 g du produit obtenu ci-dessus dans 100 $cm^3$ de chloroforme et 10 $cm^3$ de lessive de soude, refroidit à 0° + 5 °C, ajoute 25 $cm^3$ d'eau, puis goutte à goutte 4 $cm^3$ de chloroformiate de méthyle. On agite pendant 30 minutes à température ambiante, extrait au chlorure de méthylène à 10 % de méthanol, lave à l'eau saturée en chlorure de sodium, sèche, évapore à sec, et obtient 11,8 g d'un produit utilisé tel quel pour le stade suivant. F = 200 °C.

b) Réduction

On met en suspension 11,7 g d'hydrure d'aluminium-lithium dans 250 $cm^3$ de tétrahydrofuranne, refroidit à 0°-5 °C, ajoute en 10 minutes, 11,7 g du produit obtenu ci-dessus en solution dans 250 $cm^3$ de tétrahydrofuranne, après deux heures de reflux, ajoute 250 $cm^3$ de tétrahydrofuranne à 20 % d'eau, extrait au chlorure de méthylène, lave, sèche, évapore à sec et obtient 10 g de produit brut.

On dissout le produit brut ci-dessus dans 100 $cm^3$ de chlorure de méthylène, agite pendant 20 minutes avec du charbon actif, filtre, rince au chlorure de méthylène, ramène le volume à environ 100 $cm^3$, ajoute 100 $cm^3$ d'éther isopropylique, laisse cristalliser 30 minutes à température ambiante, 30 minutes au bain de glace et obtient 5,5 g du produit attendu. F = 148 °C.

Stade C : Fumarate neutre du trans 4RS 4-méthylamino 1,2,2a,3,4,5-hexahydro benz(c,d) indol-5-ol

On hydrogène 5,15 g du produit obtenu ci-dessus dissous dans 150 $cm^3$ d'acide acétique, en présence de 1,25 g de palladium à 10 % sur charbon, élimine le catalyseur par filtration, et évapore à sec. On dissout le résidu dans l'eau, neutralise à l'ammoniaque, sature de carbonate de potassium, extrait au chlorure de méthylène à 10 % de méthanol, sèche, évapore à sec et obtient 3,5 g d'un résidu que l'on dissout dans le minimum de chlorure de méthylène à 10 % de méthanol, ajoute de l'éther isopropylique, laisse cristalliser pendant une heure et obtient 2,72 g du produit attendu. F = 214-216 °C.

Formation du fumarate neutre

On met en suspension 2,7 g de base ci-dessus dans 30 $cm^3$ de méthanol et 20 $cm^3$ de chlorure de méthylène, ajoute 1,5 g d'acide fumarique, agite pendant deux heures, filtre, rince au méthanol glacé, à l'éther, recristallise dans 40 $cm^3$ d'eau et obtient 1,59 g de produit attendu. F = 304 °C.

Analyse : pour $(C_{12}H_{16}N_2O)_2$ $C_4H_4O_4$ = 524,62
Calculé : C 64,10 H 6,92 N 10,68 %
Trouvé : C 64,0 H 6,9 N 10,6 %.

Exemple 3 : Fumarate neutre de trans 4RS 4-(éthylamino) 1,2,2a,3,4,5-hexahydro benz(c,d) indol-5-ol

Stade A : Trans 4RS 4-éthylamino-1-(phénylméthyl) 1,2,2a,3,4,5-hexahydro benz(c,d) indol-5-ol

On hydrogène 10 g du produit obtenu au stade A de l'exemple 2, dissous dans 800 $cm^3$ d'éthanol, en présence de 7 g de palladium sur charbon à 9,6 % et 2,2 $cm^3$ d'acétaldéhyde après absorption de l'hydrogène, filtre le catalyseur, évapore à sec, purifie par chromatographie sur silice (éluant : chloroforme-méthanol 8/2) et obtient 5,3 g du produit attendu.
F ≃ 156 °C.

Stade B : Fumarate neutre de trans 4RS 4-(éthylamino) 1,2,2a,3,4,5-hexahydro benz(c,d) indol-5-ol

On hydrogène 5,28 g du produit obtenu ci-dessus dissous dans 400 $cm^3$ d'acide acétique, en présence de 5 g de palladium à sec. On reprend le résidu à l'eau, neutralise à l'ammoniaque, extrait au chlorure de méthylène à 10 % de méthanol, lave à l'eau la phase organique, sèche, amène à sec, redissout dans 50 $cm^3$ d'un mélange chloroforme/méthanol (1/1), ajoute en 10 minutes, 100 $cm^3$ d'éther isopropylique, laisse cristalliser une heure et obtient 1,8 g de base. F ≃ 156 °C.

Formation du fumarate neutre

On dissout 1,76 g de base dans 70 $cm^3$ de méthanol, ajoute 465 mg d'acide fumarique, laisse cristalliser une heure à température ambiante, filtre, rince au méthanol puis à l'éther et obtient 1,63 g du produit brut. F > 264 °C (avec décomposition), que l'on dissout dans l'eau au reflux, concentre à ≃ 20 $cm^3$, ajoute de l'acétone, goutte à goutte, et laisse cristalliser. On obtient le produit attendu. F = 290 °C (avec décomposition).

Analyse : pour $(C_{13}H_{18}N_2O)_2$ $C_4H_4O_4$ = 504,63
Calculé : C 65,20 H 7,29 N 10,14 %
Trouvé : C 65,3 H 7,5 N 10,2 %.

En opérant selon un procédé analogue à celui décrit ci-dessus pour la préparation de l'exemple n° 3, on a préparé les produits suivants :

| Exemples n° | 4 | | 5 | |
|---|---|---|---|---|
| $R_1$ .............. | $-CH_2-CH_2-CH_3$ | | 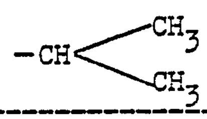 | |
| Réactif de départ | Propionaldéhyde | | Acétone | |
| Sel ............ | Fumarate neutre | | Fumarate neutre | |
| Solvant de recristallisation | Méthanol/éther isopropylique | | Chlorure de méthylène /méthanol 8/2 | |
| P F en ° C .... | 265° C | | 277° C | |
| Formule brute ... | $C_{32}H_{44}N_4O_6$ | | $C_{32}H_{44}N_4O_6$ | |
| P M ........... | 580,742 | | 580,742 | |
| Microanalyse .... Calculé/trouvé .. | | | | |
| C % | 66.18 | 66.2 | 66,18 | 66,4 |
| H % | 7,64 | 7,7 | 7,64 | 7,6 |
| N % | 9,65 | 9,7 | 9,65 | 9,4 |

Exemple 5

Le réducteur utilisé pour la condensation de l'acétone avec l'amino-alcool au stade A de l'exemple 2, est réalisé à l'aide de cyanoborohydrure de sodium (10 g pour 6,6 $cm^3$ d'acétone).

Exemple 6 : trans 4RS 4-amino 1,3,4,5-tétrahydro benz(c,d) indol-5-ol

Stade A : Trans 4RS N-/5-hydroxy 1,2,2a,3,4,5-hexahydro 1-(phénylméthyl) benz(c,d) indol-4-yl/trifluoroacétamide

On dissout sous atmosphère inerte, 10 g de produit obtenu au stade A de l'exemple 2 dans 250 $cm^3$ de chloroforme, ajoute goutte à goutte, 15 $cm^3$ d'anhydride trifluoroacétique, agite pendant une heure, distille à sec sans chauffer, sous pression réduite, ajoute 150 $cm^3$ d'eau, agite pendant 15 minutes et essore. On agite le résidu solide pendant deux heures avec 150 $cm^3$ de méthanol et 200 $cm^3$ d'une solution aqueuse saturée de bicarbonate de sodium, extrait au chlorure de méthylène, lave à l'eau, sèche, amène à sec sous pression réduite et obtient 12,5 g de produit brut. On reprend avec quelques $cm^3$ d'éther, essore, sèche pendant une heure en étuve à 50 °C sous pression réduite et obtient 9 g du produit attendu.
F = 185 °C.

Stade B : Trans 4RS N-/5-hydroxy 1,2,2a,3,4,5-hexahydro benz(c,d) indol-4-yl/trifluoroacétamide

On hydrogène 9,2 g du produit ci-dessus dans 300 $cm^3$ d'acide acétique en présence de 2,3 g de palladium à 10 % de charbon, agite pendant deux heures, filtre le catalyseur, lave à l'acide acétique et amène à sec sous pression réduite. On ajoute 200 $cm^3$ d'eau, neutralise au carbonate de sodium, extrait au chlorure de méthylène à 10 % de méthanol, lave à l'eau, sèche, amène à sec sous pression réduite, reprend le résidu avec quelques $cm^3$ d'éther, essore, sèche sous pression réduite et obtient 6,06 g du produit attendu. F = 228 °C.

Stade C : Trans 4RS N/5-hydroxy 1,3,4,5-tétrahydro benz(c,d) indol-4-yl/trifluoroacétamide

On agite pendant 16 heures sous atmosphère inerte 5,4 g du produit ci-dessus dans 550 $cm^3$ de chlorure de méthylène avec 21,6 g de bioxyde de manganèse, filtre, lave au chlorure de méthylène, amène à sec sous pression réduite, purifie par chromatographie sur silice (éluant : chloroforme-méthanol 95-5) et obtient 3,4 g du produit attendu. F = 186-188 °C.

Stade D : Trans 4RS 4-amino 1,3,4,5-tétrahydro benz(c,d) indol-5-ol

On agite sous atmosphère inerte 3 g du produit ci-dessus dans 50 $cm^3$ de soude 2N et 20 $cm^3$ de méthanol pendant deux heures, extrait plusieurs fois au chlorure de méthylène à 10 % de méthanol, lave à l'eau saturée en chlorure de sodium, sèche, filtre, amène à sec sous pression réduite et obtient 1,85 g de produit brut. On purifie le produit brut par empâtage avec du chlorure de méthylène, cristallisation dans l'isopropanol, puis dans l'éthanol et obtient 1,39 g du produit attendu. F = 182 °C.

Analyse : $C_{11}H_{12}N_2O$ = 188,22

| | | | |
|---|---|---|---|
| Calculé : | C 70,18 | H 6,43 | N 14,00 % |
| Trouvé : | C 69,2 | H 6,9 | N 14,0 %. |

Exemple 7 : Chlorhydrate de trans 4RS 4-(méthylamino) 1,3,4,5-tétrahydro benz(c,d) indol-5-ol

Stade A : Trans 4RS N-méthylamino/5-hydroxy 1,2,2a,3,4,5-hexahydro 1-(phénylméthyl) benz(c,d) indol-4-yl/trifluoroacétamide

On dissout 23 g du produit obtenu en fin du stade B de l'exemple 2 dans 1 200 $cm^3$ de chlorure de méthylène, refroidit à 0°/5 °C, ajoute, goutte à goutte, 34 $cm^3$ d'anhydride trifluoroacétique, agite pendant 2 heures à 10°/15 °C, et, amène à sec, sous pression réduite sans chauffer.

On dissout le résidu ainsi obtenu dans 400 $cm^3$ de méthanol et 200 $cm^3$ de solution saturée de bicarbonate de sodium, agite pendant 1 heure 30 minutes, extrait plusieurs fois au chlorure de méthylène, lave à l'eau, sèche, amène à sec sous pression réduite, purifie par chromatographie sur silice (éluant : benzène-acétate d'éthyle 9/1) et obtient 18 g du produit attendu F ≃ 148 °C.

Stade B : Trans 4RS N-méthylamino/5-hydroxy 1,2,2a,3,4,5-hexahydro benz(c,d) indol-4-yl/trifluoroacétamide

On hydrogène pendant une heure, 780 mg du produit ci-dessus dans 30 $cm^3$ d'acide acétique en présence de 195 mg de palladium à 10 % sur charbon, filtre ensuite le catalyseur, rince à l'acide acétique,

amène à sec à moins de 40 °C sous pression réduite, ajoute 100 $cm^3$ d'eau, neutralise à l'aide d'une solution aqueuse de bicarbonate de sodium, extrait à plusieurs reprises au chlorure de méthylène, lave à l'eau, sèche et amène à sec sous pression réduite.

On reprend le résidu avec quelques $cm^3$ d'éther, essore, recristallise dans le chlorure de méthylène et obtient 390 mg du produit attendu. F ≃ 200 °C.

Stade C : chlorhydrate de trans 4RS 4-méthylamino 1,3,4,5-tétrahydro benz(c,d) indol-5-ol

On agite pendant 16 heures sous atmosphère inerte 8,6 g de produit tel qu'obtenu ci-dessus dans 1 600 $cm^3$ de chlorure de méthylène, avec 34,4 g de bioxyde de manganèse, filtre, rince au chlorure de méthylène, amène à sec sous pression réduite, reprend le résidu par un mélange benzène-acétate d'éthyle (7/3) et isole 5,6 g de (4RS, trans) N-méthylamino (5-hydroxy 1,3,4,5-tétrahydro benz(c,d) indol-4-yl trifluoroacétamide.

On agite le produit ci-dessus dans 60 $cm^3$ de méthanol et 30 $cm^3$ de soude 2N pendant une heure, extrait au chlorure de méthylène, lave à l'eau, sèche, amène à sec sous pression réduite, obtient 3,95 g de produit brut, purifie par chromatographie sur silice (éluant : chloroforme-méthanol 5/5) et obtient 3,3 g de la base du produit attendu. F ≃ 135 °C.

Formation du chlorhydrate

On dissout à chaud, 1,5 g de base ci-dessus dans 50 $cm^3$ de chlorure de méthylène, ajoute à température ambiante 5 $cm^3$ d'éther chlorhydrique 4N, essore, lave à l'éther, sèche, purifie par recristallisation dans l'éthanol à chaud et obtient 1,46 g du produit attendu. F ≃ 240 °C.

Analyse : pour $C_{12}H_{15}N_2OCl$ = 238,71

| | | | | |
|---|---|---|---|---|
| Calculé : | C 60,37 | H 6,33 | N 11,74 | Cl 14,85 % |
| Trouvé : | C 60,2 | H 6,3 | N 11,4 | Cl 15,1 %. |

Exemple 8 : Chlorhydrate de trans 4RS 4-(éthylamino) 1,3,4,5-tétrahydro benz(c,d) indol-5-ol

On dissout 1,78 g de trans 4RS 4-(éthylamino) 1,2,2a,3,4,5-hexahydro benz(c,d) indol-5-ol dans 177 $cm^3$ de tétrahydrofuranne, ajoute sous gaz inerte, en 2 heures 30 minutes, 4,375 g d'anhydride phényl sélinique. On agite ensuite une heure, dilue avec une solution aqueuse saturée de bicarbonate de sodium puis avec de l'acétate d'éthyle, décante la phase organique, la lave à l'eau, la sèche et évapore le solvant. On chromatographie le résidu sur silice en éluant au mélange acétate d'éthyle-méthanol-ammoniaque (92-5-3) et obtient 1,451 g de produit attendu (base).

Formation du chlorhydrate

On dissout le produit dans 140 $cm^3$ d'acétate d'éthyle, ajoute lentement 2,5 $cm^3$ d'une solution de gaz chlorhydrique dans l'acétate d'éthyle ≃ 2,6N, essore le chlorhydrate et le sèche. On obtient 1,385 g de produit attendu. F = 229 °C. On purifie le produit par dissolution à chaud dans le méthanol et précipitation par addition d'éther isopropylique. F = 239 °C.

Analyse : $C_{13}H_{17}ON_2Cl$ = 252,74

| | | | | |
|---|---|---|---|---|
| Calculé : | C 61,77 | H 6,78 | N 11,08 | Cl 14,02 % |
| Trouvé : | C 61,6 | H 6,8 | N 10,9 | Cl 14,0 %. |

Exemple 9 : Chlorhydrate de trans 4RS 4-(propylamino) 1,3,4,5-tétrahydro benz(c,d) indol-5-ol

On porte à 80 °C sous gaz inerte, une solution de 4,4 g de trans 4RS 4-(propylamino) 1,2,2a,3,4,5-hexahydrobenz(c,d) indol-5-ol dans 440 $cm^3$ de xylène, ajoute rapidement 1,1 g de palladium sur charbon actif à 10 % puis porte au reflux pendant 30 minutes. On refroidit, essore, lave au méthanol et concentre le filtrat à sec. On chromatographie le résidu sur silice en éluant au mélange acétate d'éthyle-méthanol-ammoniaque (95-3-2) et obtient 3,4 g de produit attendu (base).

Formation du chlorhydrate

On dissout le produit obtenu ci-dessus dans 100 $cm^3$ d'acétate d'éthyle, puis ajoute lentement 3,9 $cm^3$ d'une solution 4,6N de gaz chlorhydrique dans l'acétate d'éthyle. On essore le chlorhydrate formé et le sèche. On obtient 3,26 g de produit attendu. F = 199 °C.

Analyse : $C_{14}H_{19}N_2OCl$ = 266,76

| | | | | |
|---|---|---|---|---|
| Calculé : | C 63,03 | H 7,17 | N 10,50 | Cl 13,29 % |
| Trouvé : | C 63,1 | H 7,2 | N 10,3 | Cl 13,3 %. |

Exemple 10 : Chlorhydrate de trans 4RS 4-(isopropylamino) 1,3,4,5-tétrahydro benz(c,d) indol-5-ol

On opère de manière analogue à celle décrite à l'exemple 9, en utilisant au départ 10,8 g de trans 4RS

4-(isopropylamino) 1,2,2a,3,4,5-hexahydrobenz(c,d) indol-5-ol. On obtient 6,18 g de chlorhydrate attendu que l'on recristallise dans l'éthanol par précipitation à l'éther isopropylique. F = 240 °C.

Analyse : $C_{14}H_{19}N_2OCl$ = 266,76

Calculé : C 63,03 H 7,17 N 10,50 Cl 13,29 %

Trouvé : C 63,3 H 7,3 N 10,4 Cl 13,3 %.

Exemple 11

On a préparé des comprimés répondant à la formule :

fumarate neutre de trans 4RS 4-(méthylamino) 1,2,2a,3,4,5-hexahydro benz(c,d) indol-5-ol 10 mg
excipient q.s. pour un comprimé terminé à 100 mg

(Détail de l'excipient : lactose, amidon, talc, stéarate de magnésium).

Exemple 12

On a préparé des comprimés répondant à la formule :

chlorhydrate de trans 4RS 4-méthylamino 1,3,4,5-tétrahydrobenz(c,d) indol-5-ol 10 mg
excipient q.s. pour un comprimé terminé à 100 mg

(Détail de l'excipient : lactose, amidon, talc, stéarate de magnésium).

Etude pharmacologique

1) Détermination de l'activité hypotensive

L'activité hypotensive a été étudiée sur des rats mâles de souche WISTAR pesant 300 g environ et anesthésiés au nembutal (50 mg/kg par voie intraveineuse).

Le produit testé a été administré par voie intraveineuse dans la veine jugulaire.

La pression artérielle càrotidienne a été mesurée avant et après administration du produit testé.

Le tableau ci-après indique les variations exprimées en pourcentage de la pression artérielle après administration du produit testé par rapport à la pression artérielle témoin initiale.

| Produits de l'exemple | Dose mg/kg | Variation % de la pression artérielle | | | |
|---|---|---|---|---|---|
| | | 1 minute après l'adminis-tration | 5 minutes après l'adminis-tration | 10 minutes après l'adminis-tration | 30 minutes après l'adminis-tration |
| 1 | 10 | - 45 | - 43 | - 40 | - 35 |
| | 1 | - 27 | - 31 | - 25 | - 27 |
| 2 | 1 | - 52 | - 48 | - 48 | - 45 |
| | 0,1 | - 65 | - 13 | - 14 | - 19 |
| 3 | 1 | - 37 | - 30 | - 29 | - 28 |
| | 0,1 | - 28 | - 11 | - 15 | - 9 |
| 4 | 1 | - 36 | - 21 | - 20 | - 15 |
| | 0,1 | - 6 | - 6 | - 6 | - 10 |
| 5 | 10 | - 35 | - 27 | - 26 | - 23 |
| | 1 | - 20 | - 16 | - 14 | - 11 |
| 7 | 1 | - 41 | - 35 | - 32 | - 32 |
| | 0,1 | - 16 | - 6 | - 6 | - 14 |
| 8 | 0,1 | - 35 | - 20 | - 16 | -13 |
| 9 | 1 | - 30 | - 40 | - 30 | -30 |
| 10 | 1 | - 49 | - 30 | - 32 | - 24 |

2) Détermination de l'activité antihypertensive

L'action antihypertensive a été étudiée sur des rats mâles spontanément hypertendus (souche Okamoto) âgés de 20 semaines pesant 300 g à 320 g.

Le produit a été administré par voie orale 48 heures après la pose d'un cathéter intracarotidien.

La pression artérielle a été mesurée en reliant le cathéter à un capteur de pression relié à un enregistreur. La pression a été mesurée avant et une heure, 4 heures et 24 heures après l'administration du produit.

Le tableau ci-après indique les variations exprimées en pourcentages de la pression artérielle, après administration du produit, par rapport à la pression témoin initiale.

| Produit de l'exemple | Dose mg/kg | Variation % de la pression artérielle | | |
|---|---|---|---|---|
| | | 1 heure après l'administration | 4 heures après l'administration | 24 heures après l'administration |
| 1 | 1 | - 7 | - 6 | - 8 |
| 2 | 1 | - 31 | - 19 | - 3 |
| | 0,1 | - 5 | - 7 | - 2 |
| 7 | 5 | - 20 | - 23 | - 18 |
| | 1 | - 4 | - 3 | - 1 |

3) Action antihypertensive chez le chien éveillé

L'étude de l'action antihypertensive du produit de l'exemple 1 a été effectuée sur le chien Beagle pesant de 12 kg à 14 kg, rendu hypertendu par enveloppement des 2 reins par de la cellophane selon la technique décrite par Irvine H. Page dans Science (1939) 89 p. 273-274.

Le produit a été administré per os aux doses de 0,1 mg/kg et 1 mg/kg.

La pression artérielle a été mesurée à la queue du chien au moyen d'un manchon pneumatique et à l'aide d'un transducteur piézo-électrique de pression. La pression a été mesurée avant, et 1 heure, 3 heures, 6 heures et 24 heures après l'administration du produit.

Le tableau ci-après indique les variations exprimées en pourcentages de la pression artérielle, après administration du produit, par rapport à la pression témoin initiale.

| Produit de l'exemple | Dose mg/kg | Variation % de la pression artérielle | | | |
|---|---|---|---|---|---|
| | | 1 er jour | | | |
| | | 1 heure après l'administration | 3 heures après l'administration | 6 heures après l'administration | 24 heures après l'administration |
| 2 | 1 | - 36 | - 42 | - 38 | - 10 |
| | 0,1 | - 24 | - 27 | - 12 | - 4 |
| 8 | 1 | - 2 | - 10 | - 11 | - 15 |
| 9 | 1 | - 27 | - 20 | - 20 | - 4 |
| 10 | 1 | - 27 | - 33 | - 14 | + 1 |

4) Etude de la toxicité aiguë

On a évalué les doses létales $DL_0$ des différents composés testés après administration par voie orale chez la souris.

On appelle $DL_0$ la dose maximale ne provoquant aucune mortalité en 8 jours. Les résultats obtenus sont les suivants :

| Produits de l'exemple | $DL_0$ en mg/kg |
|---|---|
| 1 | > 400 |
| 2 | > 800 |
| 3 | > 400 |
| 4 | 200 |
| 5 | 200 |
| 7 | > 400 |
| 8 | 80 |
| 9 | 60 |
| 10 | 100 |

**Revendications**

1. Les dérivés racémiques ou dédoublés du trans 4-amino benz(c,d) indol-5-ol, ainsi que leurs sels d'addition avec les acides minéraux ou organiques, répondant à la formule générale I :

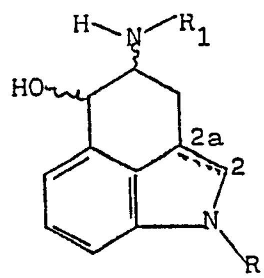

(I)

dans laquelle $R_1$ représente :

un atome d'hydrogène ;

un radical alcoyle, linéaire ou ramifié renfermant de 1 à 8 atomes de carbone, éventuellement substitué par un ou plusieurs atomes d'halogène, par un ou plusieurs radicaux hydroxy ou par un radical phényle ou phénoxy éventuellement substitué sur le noyau phényle par un ou plusieurs atomes d'halogène ou par un ou plusieurs radicaux hydroxy, méthyl ou méthoxy,

un radical cycloalcoyle renfermant de 3 à 7 atomes de carbone,

un radical cycloalcoylalcoyle renfermant de 4 à 7 atomes de carbone, ou

un radical alcényle ou alcynyle renfermant de 3 à 6 atomes de carbone, le trait pointillé en 2-2a représente une éventuelle seconde liaison carbone-carbone et R représente un atome d'hydrogène.

2. Les dérivés répondant à la formule I telle que définie à la revendication 1 dans laquelle une simple liaison est présente en 2-2a ainsi que leurs sels d'addition avec les acides minéraux ou organiques.

3. Les dérivés répondant à la formule I telle que définie à la revendication 1 dans laquelle une double liaison est présente en 2-2a ainsi que leurs sels d'addition avec les acides minéraux ou organiques.

4. Les dérivés selon la revendication 1, 2 ou 3, caractérisés en ce que dans la formule I, $R_1$ représente un atome d'hydrogène ou un radical alcoyle, linéaire ou ramifié, renfermant de 1 à 6 atomes de carbone éventuellement substitués ainsi que leurs sels d'addition avec les acides minéraux ou organiques.

5. Les dérivés selon la revendication 4, caractérisés en ce que $R_1$ représente un atome d'hydrogène ou un radical alcoyle linéaire ou ramifié non substitué, renfermant de 1 à 6 atomes de carbone, ainsi que leurs sels d'addition avec les acides minéraux ou organiques.

6. Le trans 4RS 4-méthylamino 1,2,2a,3,4,5-hexahydro benz(c,d) indol-5-ol, ainsi que ses sels d'addition avec les acides minéraux ou organiques,

7. Le trans 4RS 4-méthylamino 1,3,4,5-tétrahydro benz(c,d) indol-5-ol, ainsi que ses sels d'addition avec les acides minéraux ou organiques.

8. Procédé de préparation des dérivés du trans 4-amino benz(c,d) indol-5-ol ainsi que de leurs sels d'addition avec les acides minéraux ou organiques, tels que définis par la formule I de la revendication 1, caractérisé en ce que l'on réduit un produit de formule II :

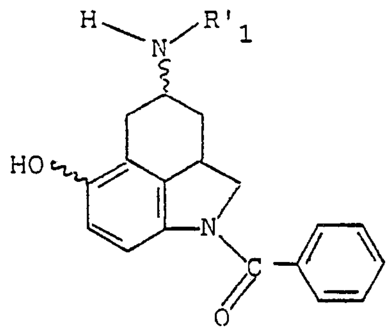

(II)

dans laquelle $R'_1$ représente un atome d'hydrogène ou un radical méthyle, pour obtenir un produit de formule $I'_A$ :

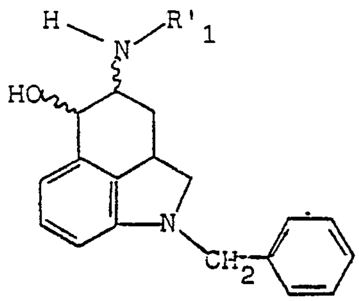

($I'_A$)

dans laquelle $R'_1$ a la signification déjà indiquée, que :

soit l'on soumet à une débenzylation, pour obtenir un produit de formule $I_A$ :

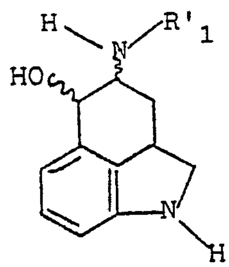

($I_A$)

dans laquelle $R'_1$ a la signification déjà indiquée, que ou bien l'on isole ou bien l'on fait réagir dans le cas où $R'_1$ représente un atome d'hydrogène, avec un dérivé carbonylé de formule III :

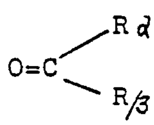

(III)

dans laquelle Rα et Rβ sont tels que

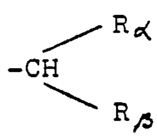

a la signification de $R_1$ déjà indiquée, à l'exception d'un atome d'hydrogène et d'un radical méthyle, puis soumet le dérivé obtenu à l'action d'un agent de réduction, pour obtenir un produit de formule $I_B$ :

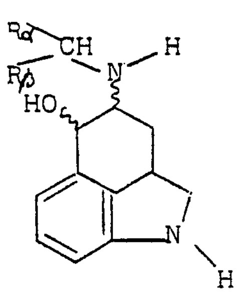

($I_B$)

dans laquelle $R_\alpha$ et $R_\beta$ ont la signification déjà indiquée, que l'on isole, si désiré,

soit l'on soumet ledit produit de formule $I'_A$ à l'action d'un agent de blocage de la fonction amine, pour obtenir un produit de formule IV :

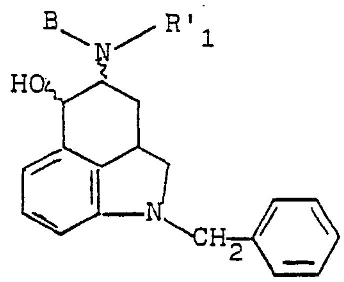

(IV)

dans laquelle B représente le groupement de blocage de la fonction amine et $R'_1$ a la signification déjà indiquée, que l'on soumet à une débenzylation, pour obtenir un produit de formule V :

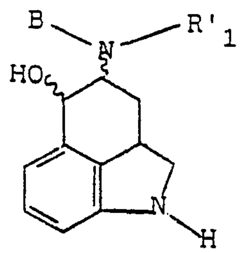

(V)

dans laquelle B et $R'_1$ ont la signification déjà indiquée, que l'on soumet à l'action d'un agent d'oxydation, pour obtenir un produit de formule VI :

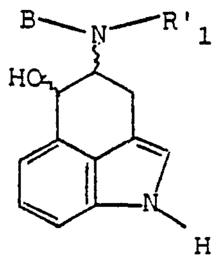

(VI)

dans laquelle B et $R'_1$ ont la signification déjà indiquée, dont on libère la fonction amine bloquée, pour obtenir un produit de formule $I_C$ :

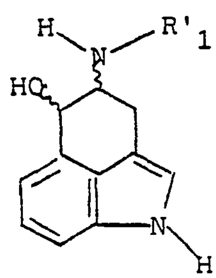

($I_C$)

dans laquelle $R'_1$ a la signification déjà indiquée, que soit l'on isole, soit l'on fait réagir, dans le cas où $R'_1$ représente un atome d'hydrogène, avec un dérivé carbonylé de formule III définie ci-dessus, puis avec un agent réducteur, pour obtenir un produit de formule $I_D$ :

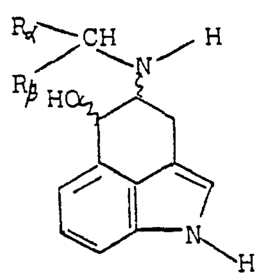

($I_D$)

dans laquelle $R_\alpha$ et $R_\beta$ ont la signification déjà indiquée ;

soit l'on fait réagir ledit produit de formule $I'_A$ dans laquelle $R'_1$ représente un atome d'hydrogène avec un dérivé carbonylé de formule III définie ci-dessus, puis avec un agent réducteur, pour obtenir un produit de formule $I'_B$ :

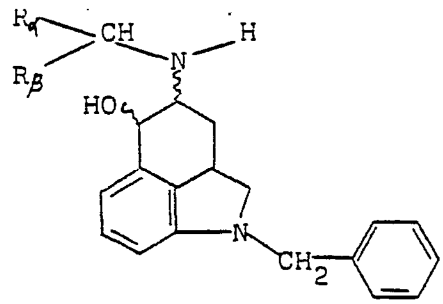

($I'_B$)

dans laquelle $R_\alpha$ et $R_\beta$ ont la signification déjà indiquée, puis, si désiré, salifie les produits de formule I obtenus au cours du procédé.

9. Procédé selon la revendication 8, caractérisé en ce que l'on débenzoyle directement le produit de formule II, pour obtenir le produit de formule $I_A$.

10. Procédé selon la revendication 8, caractérisé en ce que le groupement de blocage de la fonction amine est un radical trifluoroacétyle.

11. A titre de médicaments, les dérivés de formule I' :

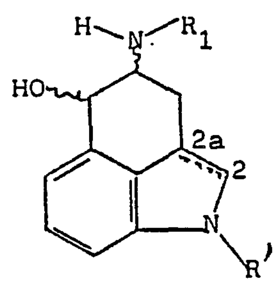

(I')

dans laquelle $R_1$ représente :

un atome d'hydrogène ;

un radical alcoyle, linéaire ou ramifié renfermant de 1 à 8 atomes de carbone, éventuellement substitué par un ou plusieurs atomes d'halogène, par un ou plusieurs radicaux hydroxy ou par un radical phényle ou phénoxy éventuellement substitué sur le noyau phényle par un ou plusieurs atomes d'halogène ou par un ou plusieurs radicaux hydroxy, méthyl ou méthoxy.

un radical cycloalcoyle renfermant de 3 à 7 atomes de carbone,

un radical cycloalcoylalcoyle renfermant de 4 à 7 atomes de carbone, ou

un radical alcényle ou alcynyle renfermant de 3 à 6 atomes de carbone,

et soit le trait pointillé en 2-2a représente une seconde liaison carbone-carbone et R' représente un atome d'hydrogène, soit le trait pointillé en 2-2a ne représente pas une seconde liaison carbone-carbone et R' représente un atome d'hydrogène ou un adical benzyle, étant entendu que lorsque R' représente un radical benzyle, $R_1$ ne représente pas un atome d'hydrogène ainsi que leurs sels d'addition avec les acides minéraux ou organiques pharmaceutiquement acceptables.

12. A titre de médicaments, les dérivés de formule I telle que définie à la revendication 2, 3, 4 ou 5, ainsi que leurs sels d'addition avec les acides minéraux ou organiques pharmaceutiquement acceptables.

13. A titre de médicaments, les dérivés de formule I tels que définis à la revendication 6 ou 7, ainsi que leurs sels d'addition avec les acides minéraux ou organiques pharmaceutiquement acceptables.

14. Compositions pharmaceutiques, caractérisées en ce qu'elles contiennent, à titre de principe actif, l'un au moins des médicaments définis à l'une des revendications 11, 12 et 13.

**Claims**

1. Racemic or resolved derivatives of trans 4-amino benz(c,d) indol-5-ol, as well as their addition salts with mineral or organic acids, answering to the general formula (I) :

(See formula page 17)

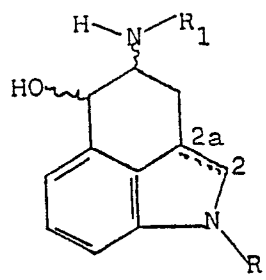

(I)

in which $R_1$ represents :

a hydrogen atom ;

a linear or branched alkyl radical containing from 1 to 8 carbon atoms, possibly substituted by one or more halogen atoms, by one or more hydroxy radicals or by a phenyl or phenoxy radical possibly substituted on the phenyl nucleus by one or more halogen atoms or by one or more hydroxy, methyl or methoxy radicals,

a cycloalkyl radical containing from 3 to 7 carbon atoms,

a cycloalkylalkyl radical containing from 4 to 7 carbon atoms, or

an alkenyl or alkynyl radical containing from 3 to 6 carbon atoms, the dotted line at 2-2a represents a possible second carbon-carbon bond and R represents a hydrogen atom.

2. Derivatives answering to the formula (I) as defined in claim 1 in which a simple bond is present at 2-2a as well as their addition salts with mineral or oganic acids.

3. Derivatives answering to the formula (I) as defined in claim 1 in which a double bond is present at 2-2a as well as their addition salts with mineral or organic acids.

4. Derivatives according to claim 1, 2 or 3, characterized in that in the formula (I), $R_1$ represents a hydrogen atom or a linear or branched alkyl radical containing from 1 to 6 carbon atoms, possibly substituted, as well as their addition salts with mineral or organic acids.

5. Derivatives according to claim 4, characterized in that $R_1$ represents a hydrogen atom or an unsubstituted linear or branched alkyl radical containing from 1 to 6 carbon atoms, as well as their addition salts with mineral or organic acids.

6. Trans 4RS 4-methylamino 1,2,2a,3,4,5-hexahydro benz(c,d) indol-5-ol, as well as its addition salts with mineral or organic acids.

7. Trans 4RS 4-methylamino 1,3,4,5-tetrahydro benz(c,d) indol-5-ol, as well as its addition salts with mineral or organic acids.

8. Preparation process for derivatives of trans 4-amino benz(c,d)-indol-5-ol as well as for their addition salts with mineral or organic acids, as defined by the formula (I) of claim 1, characterized in that a product with the formula (II) :

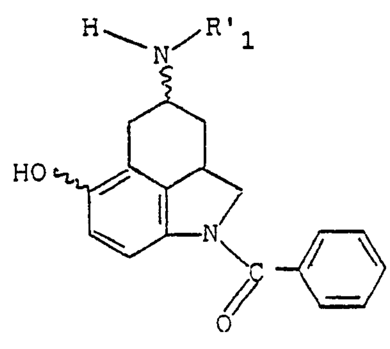

(II)

in which $R'_1$ represents a hydrogen atom or a methyl radical, is reduced, in order to obtain a product with the formula $I'_A$ :

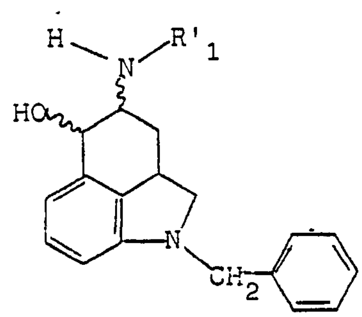

($I'_A$)

in which $R'_1$ has the significance already indicated, which :

either is submitted to a de-benzylation, in order to obtain a product with the formula ($I_A$) :

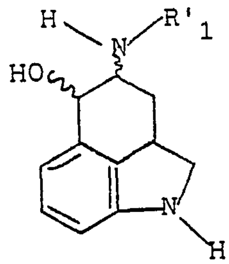

($I_A$)

in which $R'_1$ has the significance already indicated, which either is isolated or is made to react, in the case where $R'_1$ represents a hydrogen atom, with a carbonylated derivative with the formula (III) :

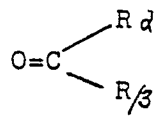

(III)

in which R alpha and R beta are such that

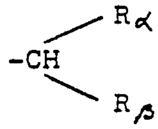

has the significance of $R_1$ already indicated, with the exception of a hydrogen atom and of a methyl radical, then the derivative obtained is submitted to the action of a reducing agent, in order to obtain a product with the formula ($I_B$) :

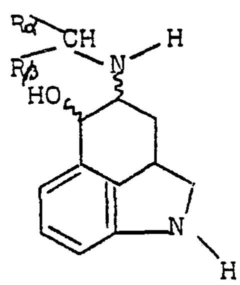

($I_B$)

in which R alpha and R beta have the significance already indicated, which is isolated, if desired,

or the said product with the formula ($I'_A$) is submitted to the action of a blocking agent of the amine function, in order to obtain a product with the formula (IV) :

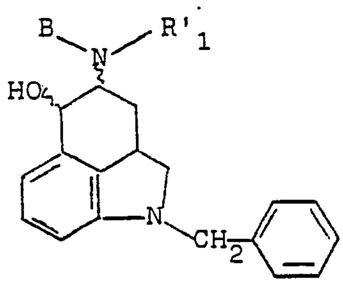

(IV)

in which B represents the blocking group of the amine function and $R'_1$ has the significance already indicated, which is submitted to a debenzylation, in order to obtain a product with the formula (V) :

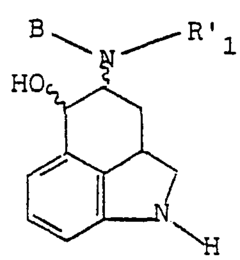

(V)

in which B and $R'_1$ have the significance already indicated, which is submitted to the action of an oxidation agent, in order to obtain a product with the formula (VI) :

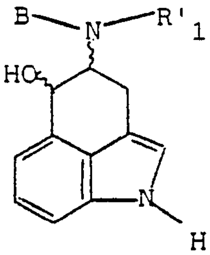

(VI)

in which B and $R'_1$ have the significance already indicated, the blocked amine function of which is freed, in order to obtain a product with the formula ($I_C$) :

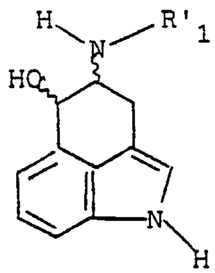

($I_C$)

in which $R'_1$ has the significance already indicated, which either is isolated, or is made to react, in the case where $R'_1$ represents a hydrogen atom, with a carbonylated derivative with the formula (III) defined above, then with a reducing agent, in order to obtain a product with the formula ($I_D$) :

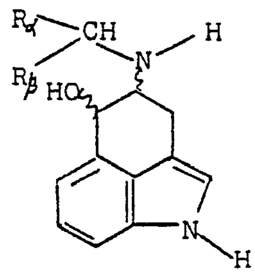

($I_D$)

in which R alpha and R beta have the significance already indicated ;

or the said product with the formula ($I'_A$) in which $R'_1$ represents a hydrogen atom is made to react with a carbonylated derivative with the formula (III) defined above, then with a reducing agent, in order to obtain a product with the formula ($I'_B$) :

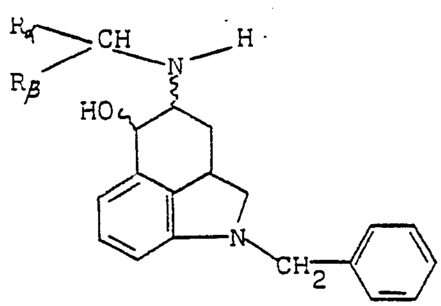

($I'_B$)

in which R alpha and R beta have the significance already indicated, then, if desired, the products with the formula (I) obtained during the process are salified.

9. Process according to claim 8, characterized in that the product with the formula (II) is directly debenzoylated, in order to obtain the product with the formula ($I_A$).

10. Process according to claim 8, characterized in that the blocking group of the amine function is a trifluoroacetyl radical.

11. As medicaments, derivatives with the formula (I') :

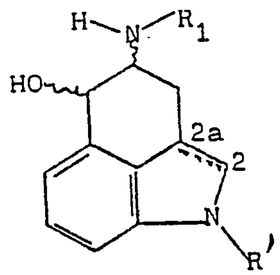

(I')

in which $R_1$ represents :

a hydrogen atom ;

a linear or branched alkyl radical containing from 1 to 8 carbon atoms, possibly substituted by one or more halogen atoms, by one or more hydroxy radicals or by a phenyl or phenoxy radical possibly substituted on the phenyl nucleus by one or more halogen atoms or by one or more hydroxy, methyl or methoxy radicals,

a cycloalkyl radical containing from 3 to 7 carbon atoms,

a cycloalkylalkyl radical containing from 4 to 7 carbon atoms, or

an alkenyl or alkynyl radical containing from 3 to 6 carbon atoms,

and either the dotted line at 2-2a represents a second carbon-carbon bond and R' represents a hydrogen atom, or the dotted line at 2-2a does not represent a second carbon-carbon bond and R' represents a hydrogen atom or a benzyl radical, it being understood that when R' represents a benzyl radical, $R_1$ does not represent a hydrogen atom — as well as their addition salts with pharmaceutically acceptable mineral or organic acids.

12. As medicaments, derivatives with the formula (I) as defined in claim 2, 3, 4 or 5, as well as their addition salts with pharmaceutically acceptable mineral or organic acids.

13. As medicaments, derivatives with the formula (I) as defined in claim 6 or 7, as well as their addition salts with pharmaceutically acceptable mineral or organic acids.

14. Pharmaceutical compositions, characterized in that they contain, as active principle, at least one of the medicaments defined in one of the claims 11, 12 and 13.

**Patentansprüche**

1. Razemische oder gespaltene trans-4-Amino-benz(c,d) indol-5-ol-Derivate sowie deren Additionssalze mit Mineral- oder organischen Säuren der allgemeinen Formel (I)

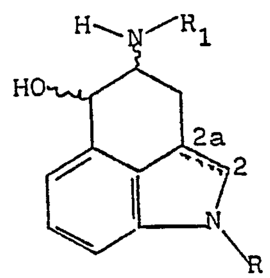

(I)

worin $R_1$ bedeutet :

ein Wasserstoffatom,

einen linearen oder verzweigten Alkylrest mit 1 bis 8 Kohlenstoffatomen, der gegebenenfalls substituiert ist durch ein oder mehrere Halogenatome, eine oder mehrere Hydroxygruppen oder einen Phenyl- oder Phenoxyrest, der gegebenenfalls an dem Phenylring durch ein oder mehrere Halogenatome oder einen oder mehrere Hydroxy-, Methyl- oder Methoxyreste substituiert ist,

einen Cycloalkylrest mit 3 bis 7 Kohlenstoffatomen,

einen Cycloalkalkylrest mit 4 bis 7 Kohlenstoffatomen oder

einen Alkenyl- oder Alkinylrest mit 3 bis 6 Kohlenstoffatomen,

wobei die gestrichelte Linie in 2-2a-Stellung eine etwaige zweite Kohlenstoff-Kohlenstoff-Bindung anzeigt und R ein Wasserstoffatom bedeutet.

2. Derivate der Formel I gemäß Anspruch 1, worin eine Einfachbindung in 2-2a-Stellung vorliegt, sowie deren Additionssalze mit Mineral- oder organischen Säuren.

3. Derivate der Formel I gemäß Anspruch 1, worin eine Doppelbindung in 2-2a-Stellung vorliegt, sowie deren Additionssalze mit Mineral- oder organischen Säuren.

4. Derivate gemäß Anspruch 1, 2 oder 3, dadurch gekennzeichnet, daß in der Formel I $R_1$ ein

Wasserstoffatom oder einen gegebenenfalls substituierten linearen oder verzweigten Alkylrest mit 1 bis 6 Kohlenstoffatomen darstellt, sowie deren Additionssalze mit Mineral- oder organischen Säuren.

5. Derivate gemäß Anspruch 4, dadurch gekennzeichnet, daß $R_1$ ein Wasserstoffatom oder einen unsubstituierten linearen oder verzweigten Alkylrest mit 1 bis 6 Kohlenstoffatomen bedeutet, sowie deren Additionssalze mit Mineral- oder organischen Säuren.

6. Trans-4RS-4-Methylamino-1,2,2a,3,4,5-hexahydro-benz(c,d)-indol-5-ol sowie dessen Additionssalze mit Mineral- oder organischen Säuren.

7. Trans-4RS-4-Methylamino-1,3,4,5-tetrahydro-benz(c,d)-indol-5-ol sowie dessen Additionssalze mit Mineral- oder organischen Säuren.

8. Verfahren zur Herstellung von trans-4-Amino-benz(c,d)-indol-5-ol-Derivaten sowie deren Additionssalzen mit Mineral- oder organischen Säuren der Formel I gemäß Anspruch 1, dadurch gekennzeichnet, daß man ein Produkt der Formel II

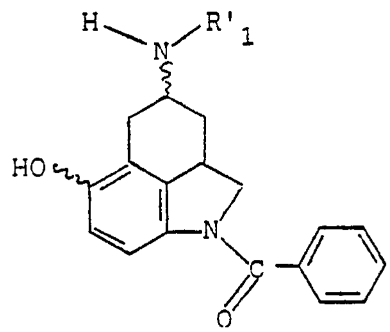

(II)

worin $R'_1$ ein Wasserstoffatom oder einen Methylrest bedeutet, reduziert, um ein Produkt der Formel $I'_A$ zu erhalten

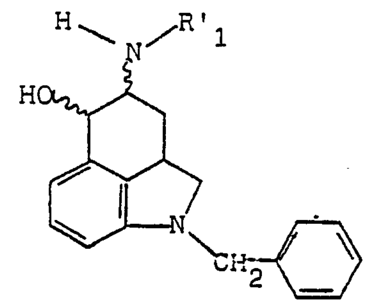

($I'_A$)

worin $R'_1$ die angegebene Bedeutung besitzt, das man

entweder einer Entbenzylierung unerzieht, um ein Produkt der Formel $I_A$ zu erhalten,

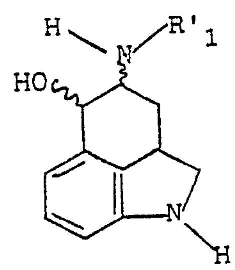

($I_A$)

worin $R'_1$ die angegebene Bedeutung besitzt, das man entweder isoliert oder, wenn $R'_1$ ein Wasserstoffatom bedeutet, mit einem Carbonylderivat der Formel III

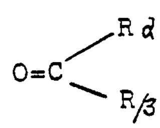

(III)

umsetzt, worin $R_\alpha$ und $R_\beta$ so beschaffen sind, daß

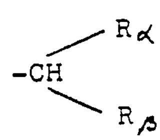

die angegebene Bedeutung von $R_1$ mit Ausnahme eines Wasserstoffatoms und eines Methylrestes besitzt, anschließend das erhaltene Derivat der Einwirkung eines Reduktionsmittels unterzieht, um ein Produkt der Formel $I_B$

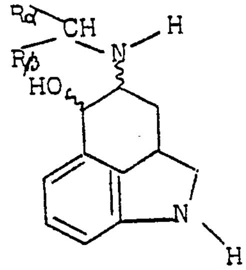

($I_B$)

zu erhalten, worin $R_\alpha$ und $R_\beta$ die angegebene Bedeutung besitzen, das man gewünschtenfalls isoliert oder das Produkt der Formel $I'_A$ der Einwirkung eines Mittels zur Blockierung der Aminfunktion unterzieht, um eine Produkt der Formel IV

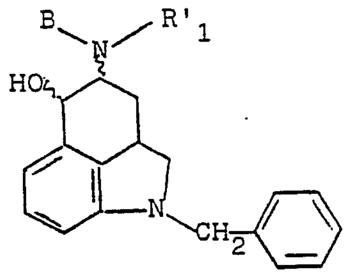

(IV)

zu erhalten, worin B die blockierende Gruppe der Aminfunktion darstellt und $R'_1$ die angegebene Bedeutung besitzt, welches man einer Entbenzylierung unterzieht, um ein Produkt der Formel V

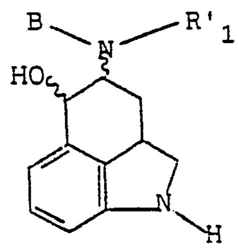

(V)

zu erhalten, worin B und $R'_1$ die angegebene Bedeutung besitzen, das man der Einwirkung eines Oxydationsmitels unterzieht, um ein Produkt der Formel VI

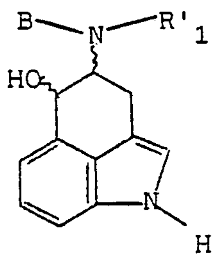

(VI)

zu erhalten, worin B und $R'_1$ die angegebene Bedeutung besitzen, dessen blockierte Aminfunktion man freisetzt, um ein Produkt der Formel $I_C$

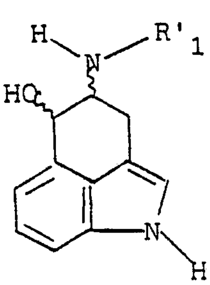

($I_C$)

zu erhalten, worin $R'_1$ die angegebene Bedeutung besitzt, welches man entweder isoliert oder, wenn $R'_1$ ein Wasserstoffatom bedeutet, mit einem Carbonylderivat der vorstehend definierten Formel III, danach mit einem Reduktionsmittel umsetzt, um ein Produkt der Formel $I_D$

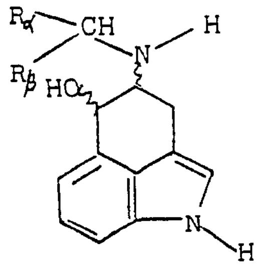

($I_D$)

zu erhalten, worin $R_\alpha$ und $R_\beta$ die angegebene Bedeutung besitzen

oder das Produkt der Formel $I'_A$, worin $R'_1$ ein Wasserstoffatom bedeutet, mit einem Carbonylderivat der vorstehend definierten Formel III, danach mit einem Reduktionsmittel umsetzt, um ein Produkt der Formel $I'_B$

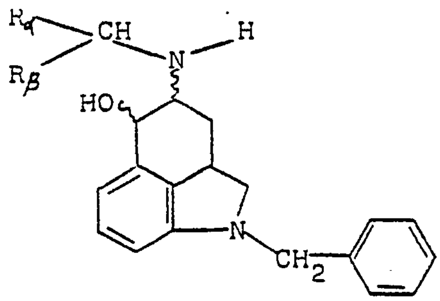

($I'_B$)

zu erhalten, worin $R_\alpha$ und $R_\beta$ die angegebene Bedeutung besitzen, und anschließend gewünschtenfalls die im Verlauf des Verfahrens erhaltenen Produkte der Formel I ein Salz überführt.

9. Verfahren gemäß Anspruch 8, dadurch gekennzeichnet, daß man das Produkt der Formel II direkt entbenzyliert, um das Produkt der Formel $I_A$ zu erhalten.

10. Verfahren gemäß Anspruch 8, dadurch gekennzeichnet, daß die Gruppe zur Blockierung der Aminfuktion eine Trifluoracetylgruppe ist.

11. Als Arzneimittel die Derivate der Formel I'

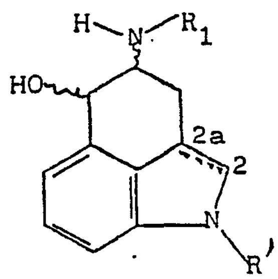

(I')

worin $R_1$ bedeutet:

ein Wasserstoffatom

einen linearen oder verzweigten Alkylrest mit 1 bis 8 Kohlenstoffatomen, der gegebenenfalls substituiert ist durch ein oder mehrere Halogenatome, eine oder mehrere Hydroxygruppen oder einen Phenyl- oder Phenoxyrest, der gegebenenfalls an dem Phenylring durch ein oder mehrere Halogenatome oder durch eine oder mehrere Hydroxy-, Methyl- oder Methoxygruppen substituiert ist,

einen Cycloalkylrest mit 3 bis 7 Kohlenstoffatomen,

einen Cycloalkalkylrest mit 4 bis 7 Kohlenstoffatomen oder

einen Alkenyl- oder Alkinylrest mit 3 bis 6 Kohlenstoffatomen und

entweder die gestrichelte Linie in 2-2a-Stellung eine zweite Kohlenstoff-Kohlenstoffbindung darstellt und R' ein Wasserstoffatom bedeutet, oder die gestrichelte Linie in 2-2a-Stellung keine zweite Kohlenstoff-Kohlenstoffbindung bedeutet, und R' ein Wasserstoffatom oder einen Benzylrest darstellt, wobei, wenn R' einen Benzylrest darstellt, $R_1$ kein Wasserstoffatom bedeutet, sowie deren Additionssalze mit pharmazeutisch verträglichen Mineral- oder organischen Säuren.

12. Als Arzneimittel die Derivate der Formel I gemäß Anspruch 2, 3, 4 oder 5 sowie deren Additionssalze mit pharmazeutisch verträglichen Mineral- oder organischen Säuren.

13. Als Arzneimittel die Derivate der Formel I gemäß Anspruch 6 oder 7 sowie deren Additionssalze mit pharmazeutisch verträglichen Mineral- oder organischen Säuren.

14. Pharmazeutische Zusammensetzungen, dadurch gekennzeichnet, daß sie als Wirkstoff zumindest eines der Arzneimittel gemäß einem der Ansprüche 11, 12 und 13 enthalten.